# EUROPEAN PATENT SPECIFICATION

(11) **EP 2 226 078 B1**
(45) Date of publication and mention of the grant of the patent: **02.09.2020**
(21) Application number: 10155558.9
(22) Date of filing: 04.03.2010
(51) Int. Cl.: A61K 36/899, A61K 31/716, A61P 31/10

(54) **Anti-fungal fermented cereal grain compositions**
Antifungizide Verbindungen und Zusammensetzungen aus fermentierte Getreide
Composés antifongiques à base de céréales fermentées

(30) Priority: 04.03.2009 NL 1036661
(43) Date of publication of application: 08.09.2010
(73) Proprietor: Serrix BV, 1001 EL Amsterdam (NL)
(72) Inventor: Brand-Garnys, Elzbieta Ewa, 2914 AD Nieuwerkerk a/d IJssel (NL)
(74) Representative: Farago-Schauer, Peter Andreas

(56) References cited:
- WO-A-03/053376
- WO-A1-2007/036230
- DE-A1- 19 537 509
- DE-U1-202006 009 610
- US-A1- 2003 119 780
- US-A1- 2007 154 581
- GUPTA A K ET AL: "Therapies for Onychomycosis: A Review" DERMATOLOGIC CLINICS, W.B. SAUNDERS CO., LONDON, GB, vol. 24, no. 3, 1 July 2006 (2006-07-01), pages 375-379, XP009119927 ISSN: 0733-8635
- BOROS LASZLO G ET AL: "Fermented wheat germ extract (Avemar) in the treatment of cancer and autoimmune diseases." ANNALS OF THE NEW YORK ACADEMY OF SCIENCES JUN 2005, vol. 1051, June 2005 (2005-06), pages 529-542, XP002537200 ISSN: 0077-8923
- Anonymous: "Finally Getting Rid of Toe Nail Fungus without Drugs - Green Health Spot", , 12 February 2008 (2008-02-12), XP055466326, Retrieved from the Internet: URL:http://www.greenhealthspot.com/2008/02 /finally-getting.html [retrieved on 2018-04-11]

## Description

### Technical field

The invention relates to compositions, particular as used for treating fungal infection and more particular for fungal infections of the skin (dermatomycosis) and most particular for nail infections (onychomycosis). Examples of fungal skin and nail infections are tinea pedis, a fungal infection of the feet principally involving the toe webs and soles, and onychomycosis, a fungal infection of the toe or finger nails.

### Background art

Several antifungal compounds in the art are indicated for fungus infections of the foot, while some can be used to treat fungal nail infections. Compounds tend to be selected for development on the basis of their broad spectrum of anti-fungal activity. Examples of such compounds include amoralfine (Curanail®), ciclopirox (Penlac®), clotrimazol, fluconazole (Diflucan®), griseoulvin, imidazoles, itraconozole (Sporanox®), ketoconazole (Nizoral®), metrimidazol, miconazol (Daktarin®), olamine 8% nail lacquer solution, sodium pyrithione, terbinafine (Lamisil®) and natural products such as tea tree oil (Ferula®, Nailer®).

Fungi located in callus and particularly on, under, or in nails are difficult to reach with medication. The thickness of the nail and its compact structure make it difficult for antifungal agents to penetrate the nail to reach the site of action. If not properly treated, the infection will return. Currently available anti-fungus compounds, such as miconazole, have low penetration of the nail. Also, topically applied terbinafine shows very limited efficacy against onychomycosis, possibly due to its nonionic character. When applied topically, they require a long treatment periods, for instance up to 9 months for topical miconazol (Daktarin®), often with low efficacy rates. WO2006/036144A1 discloses carrier-free composition comprising natural ingredients such as thymol, camphor, menthol and Eucalyptus citridiora oil for treatment of onychomycosis.

To enhance nail penetration, WO 00/15202 refers to particular C1-C4 alkyl esters penetration enhancers, while WO 02/11764 even suggests drilling holes in nails. WO2005092299 suggests a transnail patch containing an amorolfine antifungal with a penetration enhancer. US6159977 and US6391879 use dimethyl sulfoxide (DMSO) as a delivery vehicle for antifungal compounds. US6224887 discloses penetration enhancing agents for antifungal agents in nail lacquer formulations comprising film forming polymer, and volatile solvent. Gupta AK et al "Therapies for onychomycosis: a review", dermatologic clinics, W.B. Saunders Co., London, GB, vol 24 no 3, 1 July 2006, p. 375-379 provides an overview of onychomycosis treatments.

To overcome efficacy problems, systemic administration has been proposed for antifungal compounds. This, however, can lead to long treatment periods and expanded drug exposure, causing considerable side-effects and/or creating other inconveniences such as chemical or physical composition instability. For instance, anti-fungal efficacy in the nail tends to be only 15-30% for griseofulvin and ketoconazole after up to 18 months. WO96/13981 and GB 1539756 disclose fermenting cereal germs with lactic acid bacteria. EP1458334 and DE29923627 describe fermented cereal extracts and refer to usage in cosmetic formulations. US6042845 discloses a combination of sulfhydryl containing amino acid and urea to enhance permeation of antifungal drugs through nail tissue.

Surprisingly, we have found a way of improving results when treating fungus infections of the foot and particularly in the nail without the disadvantages of compounds and compositions of the prior art, including lack of efficacy (for instance in immunocompromised patients), long treatment periods, long drug exposure, undesirable side effects, and/or chemical or physical instability of the compound or composition.

### Disclosure of the invention

The present invention relates to fermented cereal grain for use in treatment of fungal infections. Another aspect that is not part of the invention relates to the use of fermented cereal grain for the manufacture of a medication for the treatment of fungus infections. Particularly, the fermented cereal grain is Lactobacillus species fermented rye. The description also relates to composition with Lactobacillus species fermented cereal grain and a penetration enhancer. Another aspect of the description is a method of preparing an antifungal composition comprising fermented cereal grain, wherein the grain is mixed with water and with a penetration enhancer. Yet another aspect of the invention relates to an applicator comprising a brush, a reservoir, a cap, and a liquid composition comprising fermented cereal grain and preferably a penetration enhancer.

Surprisingly, we have found that compounds and compositions according to the invention show increased anti-fungal efficacy in treatment of foot and particularly of nail infections.

For the purpose of this invention, anti-fungal activity is defined as having a direct anti-fungal effect. According to the invention, a direct antifungal effect is specifically defined as reducing the number of colony forming units when exposed to the antifungal as a matrix for 24 hours.

More specifically, a direct antifungal effect is defined as having a killing rate of at least log 2 of an inoculation of 10^5 CFU/g colony forming units after exposure to the antifungal product as a matrix for 24 hours. Preferably, *Trichophyton Rubrum, Trichophyton Mentagrophytus* and/or *Epidermophyton floccosum* are used as CFU. An example of a test for direct antifungal activity is shown in Example 1. By having such a direct antifungal effect, compounds and compositions of the invention, thus, do not solely work indirectly, for instance by stimulating the immune system of the host to attack the fungus. It should be understood that compounds that work indirectly, such as for instance described in WO2005027936, may not be effective in patients with a compromised immune system and, thus, are less preferred.

Contrary to popular believe, we have found that broad spectrum anti-fungal compounds of the art are not optimal for topical treatment of foot and/or nail fungus infections.

Without wishing to be bound by any theory, the broad spectrum activity might compromise the efficacy against specific fungi, while the structure might prevent reaching the site of action.

The invention provides improved clinical results by using compounds and compositions selected because of their optimal activity against specific fungi, particularly targeting *Trichophyton Rubrum, Trichophyton Mentagrophytus* and *Epidermophyton floccosum,* and even more particularly *Trichophyton Rubrum,* and *Trichophyton Mentagrophytus* which are notorious to treat.

In addition, compounds and compositions of the invention have been optimized to reach the site of action in the nail. Surprisingly, we have identified anti-fungal compounds that not only show high efficacy against the three particular fungi, but also are able to penetrate the nail. Consequently, we have surprisingly found that trans-dermal and even trans-nail transport is possible with good efficacy results, limited or no side-effects, short treatment periods and/or chemically and/or physically stable compositions.

### Anti-fungal compounds

Surprisingly, we have found that fermented cereal grain has excellent activity specifically against *Trichophyton Rubrum, Trichophyton Mentagrophytus* and *Epidermophyton floccosum,* while also showing potential to reach the site of action, e.g. in the nail. Consequently, we have surprisingly found that trans-dermal and even trans-nail transport is possible with good anti-fungal efficacy results, limited or no side-effects, short treatment periods and/or chemically and/or physically stable compositions.

US 2005180964 relates to inhibition of growth of particular infectious Aspergillus fumigatus in the gut of mammalian and avian species and uses a combination of β-1,3,(4)-endoglucanohydrolase, yeast-derived β-1,3(4) glucan, diatomaceous earth SiOx2), mineral clay, and glucomannan. WO 03/097091, US2007154581, WO2005027936, WO2006030318, and WO 2005/067977 mention use of glucans in immunostimulation. WO2004/084947 discloses that β glucans improve bioavailability of pharmaceutically active compounds in the GI tract. US6413715 discloses β-1,3-d-glucan as marker for systemic fungal infection. DE202006009610 and DE202006003249 disclose anti-fungal compositions comprising a complex of Chitin, β 1,3/1,6 D Glucan and Melanin (CGM), produced by the fungus Fomes Fomentarius. Boros Laszlo G. et al: "Fermented wheat germ extract (Avemar) in the treatment of cancer and autoimmune diseases.", annuls of the New York Academy of Sciences, June 2005, p. 529-542 discloses germ extracts for use as anti-cancer agents and for use in certain autoimmune conditions.

The anti-fungal compounds of the invention are fermented rye. The cereal grain is bacteria-fermented, the bacteria being Lactobacillus species. Examples of suitable lactobacillus are Lactobacillus DSM 6037 and Lactobacillus DSM 6129. Most preferably, fermentation takes place under conditions as specified in DE29923627U and most preferably as in EP145 8334.

A preferred compound for use in the present invention is Woresana® serum. Surprisingly, we have found that the serum contain water, β-1,3-d-glucans (average molecular weight 500 - 2,000,000 Dalton), lactic acid, panthotenic acid (vitaminB5), nicotinamide (vitamin B3), folic acid (vitamin B12), amino acids or combinations thereof. Woresana® serum is a water-based, slightly viscous, transparent solution. Woresana® is a registered trademark of Woresan GmbH. Surprisingly, we have found that β-1,3-d-glucans and/or lactic acid and/or panthotenic acid (vitaminB5) and/or nicotinamide (vitamin B3), folic acid (vitamin B12) has antifungal activity, specifically direct antifungal activity.

Without wishing to be bound by any theory, Applicants believe that fermented cereal grain, particularly fermented rye, is surprisingly effective, when topically applied, in combating foot and particularly nail fungus infections caused by the three particular fungi. Efficacy is further enhanced by combination with a penetration enhancer, particular the penetration enhancer of the present invention, as discussed below.

### Penetration enhancer

To further improve efficacy at the site of action, the present invention is in particular directed to compositions preferably comprising at least one penetration enhancer. Penetration enhancers, preferably trans-skin and more preferably trans-nail penetration enhancers, promote drug penetration through the skin and particularly through the nail. Preferably, they are safe, inert in the formulation and do not have other concerns such as intense smell or high costs. Penetration enhancer efficacy can be studied in-vitro with the Franz diffusion cell: a cell consisting of two compartments that are separated by a membrane, for instance a pig skin. A product comprising a penetration enhancer is applied to one compartment and the concentration of the product is measure in the other compartment over time.

One reason that anti-fungal compounds of the prior art combined with enhancers still show no or only limited efficacy in nail fungus treatment, might be that in-vitro results are not a substitute for positive in-vivo efficacy results. Anti-fungal compounds and compositions according to the invention however have both good in-vitro and in-vivo efficacy results without the negatives of the anti-fungal compounds of the prior art, such as side-effects, limited activity, long treatment periods and/or chemical or physical instability.

Preferably, the penetration enhancer of the invention is selected from the group of ethanol, glyceryl monoethyl ether, monoglycerides (such as glyceryl monooleate), isopropylmyristate, lauryl alcohol and derivatives (such as lauric acid, lauryl lactate), isoprenoids (such as terpineol, menthol, d-limonene, cineol, etc), beta-cyclodextrins, dimethyl sulfoxide (DMSO), Phytantriol, dimethyl isosorbide, polysorbates, fatty acids with C6-C14 (oleic acid), N-methylpyrrolidone, polyglycosylated glycerides, 1-dodecylazacycloheptan-2-one (Azone®), cyclopentadecalactone (CPE-215®), 2-(n-nonyl)-1,3-dioxolane (SEPA®), n-octanol, esters (such as oleyl oleate), decanal dimethyl acetal (DDMA), cyclopentadecalactone (CPE-215®, Bentley), and unsaturated phosphatidylcholine.

Preferably, the penetration enhancer of compositions of the invention is selected unsaturated phosphatidylcholine and dimethyl isosorbide. Most preferably the penetration enhancer is dimethyl isosorbide.

Preferably, the composition of the invention comprises from 0.1 to 20 % by weight of penetration enhancer, more preferably from 0.2 to 10% by weight. Preferably, the composition comprises from 1.5 to 6% by weight of unsaturated phosphatidylcholine. More preferably, the composition comprises from 0.5 to 2% by weight of dimethyl isosorbide.

### Solvent

Preferably, hydrophilic solvents are used in the invention. Solvent may be selected from water, C2-C10 alcohols (short chain alcohols), esters of molecular weight less than 150 Dalton (low molecular weight esters), ketones of molecular weight of less than 150 Dalton (low molecular weight ketones), hydrophilic glycols (polyethylene glycols with n=1-150, such as pentylene glycol) and polyols (such as glycerol). More preferably, the solvent is selected from water and pentylene glycol. Particularly preferred is a mixture of water and glycol, especially pentylene glycol, simultaneously enabling microbiological control.

Preferably, compositions according to the present invention comprise from 1 to 99 % by weight of solvent, more preferably from 10 to 95 % by weight, most preferably from 20 to 90 % by weight.

Preferably, compositions according to the present invention comprise from 1 to 99% by weight of solvent, more preferably from 10 to 95 % by weight.

### Thickening agent

Preferably, compositions of the invention comprise a thickening agent to facilitate easy application of the composition on the skin and particularly on the nail.

Preferably, the thickening agent is selected from the group of cellulose ethers, acrylic thickeners, natural gums, more preferably from cellulose ethers. Preferred example of thickening agents are hydroxyethylcellulose (Natrosol 250 HHX Pharm from Hercules BV), for instance at a level of 0.5% by weight.

Preferably, compositions according to the present invention comprise from 0.2 to 5 % by weight of thickening agent.

### Anti fungal compositions

Anti-fungal compositions for use according to the invention comprise an anti-fungal agent and preferably other ingredients, as pointed out above. Optional ingredients may be included.

Compositions for use according to the invention may be liquids or semi-solid flowing media, e.g. creams, gels, solutions, lotions, ointments, patches or nail varnishes. Preferably, the composition is liquid. If liquid, the antifungal composition according to the invention preferably has a Brookfield viscosity of from 100 to 20,000 cPs, more preferably from 150- 2000 cPs, most preferably from 300-1000 cPs.

In a further embodiment, the present invention relates to compositions for use in antifungal treatment comprising fermented cereal grain, penetration enhancer and PolyVinylAlcohol (PVA) polymer. Preferably, the composition comprises from 1% to 20%, more preferably from 5% 15% by weight of the polymer. We have found that these compositions are particularly suitable for treatment of foot fungus as they can be easily applied, have to good antifungal activity and excellent product qualities. Preferably, polyethyleneglycol (PEG) is added to this composition at a level of from % to 10%, more preferably from 2% to 5% by weight. Examples of suitable polyethyeleneglycol are PEG-8 and PEG-90.

Surprisingly, we have found that the PEG contributes to film formation of the PVA polymer, leading to more flexible, consumer preferred film qualities that can be more conveniently removed. Preferably, an osmosisprotector is added at a level of from 0.1 to 5%, more preferably 0.3 to 2% by weight of the composition. A suitable example is betaine. We have surprisingly found that the osmosisprotector advantageously protects the skin again the dehydration effects of the PVA polymer.

### Method of preparation

The present invention relates to a method of preparing an antifungal composition comprising fermented cereal grain, wherein the grain is mixed with water and preferably with at least one penetration enhancer. Preferably, compositions of the invention are prepared by mixing the ingredients. The thickening agent is preferably dispersed in the solvent. Preferably, the antifungal compound is subsequently added. Preferably, the penetration enhancer is subsequently added. Preparation according to the invention leads to chemically and/or physically stable compositions.

### Method of treatment

While the compositions of the present invention can be applied generally, it is preferred that they are applied topically, more preferably on the nail.

Another aspect is a Lactobacillus species fermented rye for use in treating onychomycosis by applying an antifungal composition comprising Lactobacillus species fermented rye. Preferably, the antifungal composition also comprises at least one penetration enhancer.

### Applicator

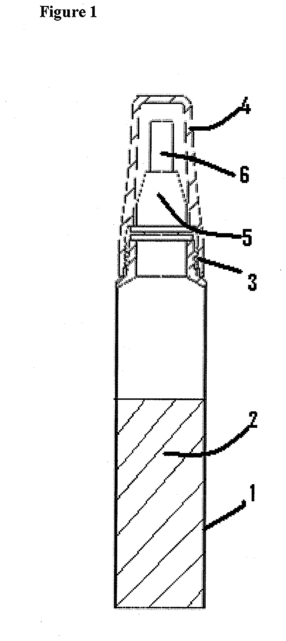

The applicator is shown in the drawing of figure 1. A brief description of Figure 1: container (1) holds the anti-fungal composition (2) of the invention; closing mechanism (3) for cap (4) is in this embodiment of the screw type (modification are possible such as clicking closures, etc.); nail and/or skin applicator (5) comprises brush (6) (modification are possible such as a plastic, stick, cotton wool, etc) which is used for application of the formulation to the nail and/or skin.

### Examples

The following Examples illustrate the invention.

### Example 1 - In-vitro activity of Woresana® serum

Three different types of agar petridishes:
1) Saboraud Dextrose agar (SDA) from Oxoid, Haarlem, The Netherlands,
2) Malt Extract Agar (MEA) from Duchefa, Haarlem, The Netherlands and,
3) Chloramphenicol gist extract glucose Agar (CGGA) from Duchefa, Haarlem, The Netherlands)
are inoculated with a culture of Trichophyton Mentagrophytes (Deutsche Sammlung von Mikroorganismen und Zellkulturen GmbH, DSMZ nr. 4870, 10³ kve/ml). Woresana® serum (batchnr. 240407) is added till concentrations of 15%, 25%, 50%, 75 and 100%. The Petri-dishes are stored at two different temperatures (30 °C and 37 °C) and the concentration of Trichophyton mentagrophytes is determined by visual counting using a microscope. The following table summarize the test results in number of colonies for diluted Woresana serum of 50%.

| test strain: DSMZ-Nr.4870 | t=0 (in days) | t=5 (in days) | T=6 (in days) | t=7 (in days) | t=8 (in days) | T=9 (in days) | T=10 (in days) |
|---|---|---|---|---|---|---|---|
| | CFU/g | CFU/g | CFU/g | CFU/g | CFU/g | CFU/g | CFU/g |
| Trichophyton mentagrophyt es (in cfu/g) | 10³ | 10³ | 10³ | 10³ | 10² | 80 | < 10 |

It can be concluded that the preparation was an effective direct antifungal composition and reduced the Trichophyton mentagrophytes culture in 10 days with a factor >100 (log 2). Similar results with obtained with cultures of *Trichophyton Rubrum* and *Epidermophyton floccosum.*

### Example 2 - Antifungal activity of formulation

14 human volunteers (male and female between 18-65 years) with a confirmed diagnosis of onychomycosis (nail fungus) caused by *Trichophyton rubrum, Trichophyton mentagrophytes* and/or *Epidermophyton floccosum* participated in a single center, open label trial. The antifungal composition was prepared as indicated:
The anti-fungal composition comprises 48% by weight of water (solvent), 10% by weight of Hydrolite-5 (pentyleneglycol; from Symrise GmbH & Co KG; solvent/diluent), 0.5% by weight of Natrosol 250 HHX Pharm (thickening agent; from Hercules BV;
hydroxyethylcellulose), 40% by weight of Woresana ® serum (Lactobacillus Rye Ferment; from Cremer Oleo; antifungal agent), and 1.5% by weight of Arlasolve DMI Dimethyl (Isosorbide; from Uniqema; penetration enhancer)

The antifungal composition was prepared by adding Hydroxyethylcellulose (Natrosol®) to the mixture of water and pentylene glycol (Hydrolite-5®) Lactobacillus species fermented rye serum (Woresana® serum) was added under stirring and, subsequently, dimethyl isosorbide (Arlasolve DMI Dimethyl®) was added under mixing. The applicator of Figure 1 was filled with the antifungal composition of the invention and the applicator was closed with the nail/skin application and the cap.

The antifungal composition of the invention was applied twice daily for four weeks with the applicator. Visits to the clinic were scheduled at day 0 (screening), day 28 and day 56, while the volunteers kept a weekly diary on application frequency and occurrence of symptoms and severity (1 excellent; 2 good; 3 moderate; 4 bad) during a period of 8 weeks. Nail clipping and skin scrapings were collected and send to the laboratory for analysis of the type and amount of fungus infection. Nail clippings were inoculated onto Malt extract + chloro. 0,1g/L agar, incubate at 30°C and analyzed. Trichophyton rubrum, Trichophyton mentagrophytes and Epidermophyton floccosum cultures were measured by counting of number of colonies, when necessary with the help of microscope.

The results can be summarized as follows:
After the 4 week treatment period with the antifungal formulation, 71% of the volunteers had no detectable nail fungus infection in the nail samples (100% reduction). In none of these volunteers, fungus infections were found after 8 weeks (56 days). Note that these volunteers did not receive treatment between week 4 and week 8 which further illustrates the remarkable efficacy of the compounds and compositions of the present invention.
The remainder (29%) of the volunteers showed detectable nail fungus infections in the nail samples after 4 weeks of treatment. However, the number of CFU in the nail samples was reduced by 84% on average.

No adverse events were recorded during the cause of the clinical trial.

Chemical and physical stability of the formulation was confirmed after storage at 25°C for 4 weeks.

It can be concluded that antifungal compounds and compositions according to the invention show excellent anti-fungal activity, require shorter treatment periods, show fewer and/or less severe side-effects and are chemically and physically stable.

### Example 3

The following formulation was prepared:

| | |
|---|---|
| Demineralised water | ad 100,0 % |
| Lactobacillus Rye Ferment | 15,0 % |
| Polyvinyl Alcohol | 10,5 % |
| Betaine | 6,0 % |
| Pentylene Glycol | 5,0 % |
| PEG-8 | 2,4 % |
| Dimethylisosorbide | 1,5 % |
| PEG-90 | 0,8 % |
| Phytantriol | 0,6 % |

Polyvinyl alcohol (PVA) was dissolved in water using a propeller mixer at 90°C, taking about 20-40 minutes 90°C and a transparent, colourless & high viscous solution is obtained, free of agglomerates. A solution is prepared of PEG-8, PEG-90 and pentylene glycol, at 35-40°C to speed up dissolution. The homogeneous low-viscous solution is heated to 70-80°C and added to the polyvinyl alcohol solution of 90°C while stirring with a planetary mixer. The transparent, homogeneous solution is cooled to 30°C Lactocaillus Rye Ferment is added, followed by betaine, dimethylisosorbide and phytantriol. The mixtures is stirred until homogeneous. Optionally solubilised fragrance and/or essential oils can be applied. Other anti-fungal products may equally be used in the formulation such as natamycin, nystatin, amphetericin, imidazole-derived fungicides such as ketoconazole, terbinafin, clotrimazole, etc., and several other products active against Trichophyton spcs.

20 to 25g of the viscous formulation is applied as a mask to the skin and allowed to dry until film formation. After 20 minutes the gel can be removed as a soft film, in one piece. Good results were obtained when using the formulation in the treatment of fungal infections of the foot (athlete's foot).

## Claims

1. Fermented cereal grain for use in the treatment of fungal infections, wherein the fermented cereal grain is Lactobacillus species fermented rye.

2. Fermented cereal grain for use according to claim 1, wherein the grain targets *Trichophyton Rubrum, Trichophyton Mentagrophytus* and *Epidermophyton floccosum*

3. Fermented cereal grain for use according to claims 1-2, wherein the fungal infection is onychomycosis.

4. Fermented cereal grain for use according to claims 1-3, wherein the fermented cereal grain is present in a composition with a penetration enhancer.

5. Fermented cereal grain for use according to claim 4, wherein the composition further comprises from 1 to 99% by weight of solvent.

6. Fermented cereal grain for use according to claims 4-5, wherein the composition further comprises a thickening agent.

7. Fermented cereal grain for use according to claims 4-6, wherein the penetration enhancer is selected from the group of ethanol, glyceryl monoethyl ether, monoglycerides, isopropylmyristate, lauryl alcohol and derivatives, isoprenoids, beta-cyclodextrins, dimethyl sulfoxide, phytantriol, dimethyl isosorbide, polysorbates, fatty acids with C6-C14, N-methylpyrrolidone, polyglycosylated glycerides, 1-dodecylazacycloheptan-2-one, cyclopentadecalactone, 2-(n-nonyl)-1,3-dioxolane, n-octanol, esters, decanal dimethyl acetal, cyclopentadecalactone, and unsaturated phosphatidylcholine.

8. Fermented cereal grain for use according to claims 4-7, wherein the composition further comprises polyvinylalcohol polymer.

9. Fermented cereal grain for use according to claims 4-8, wherein the composition further comprises an osmosis protector at a level of from 0.1 to 5% by weight of the composition.

10. Fermented cereal grain for use according to claims 1-9, wherein the fermented cereal grain is liquid and is present in an applicator with a brush, a reservoir, and a cap.

11. Fermented cereal grain for use according to claims 1-10, wherein the fermented cereal grain is applied to a nail and/or a skin.

12. Nail applicator for use in the treatment of onychomycosis comprising a brush, a reservoir, a cap, and a liquid antifungal composition comprising fermented cereal grain according to claim 1.

13. Applicator for use according to claim 12, wherein the antifungal composition further comprises a penetration enhancer.

## Patentansprüche

1. Fermentiertes Getreide zur Verwendung in der Behandlung von Pilzinfektionen, worin das fermentierte Getreide ein mit Lactobacillus-Spezies fermentierter Roggen ist.

2. Fermentiertes Getreide zur Verwendung gemäß Anspruch 1, worin die Targets des Getreides *Trichophyton Rubrum, Trichophyton Mentagrophytus* und *Epidermophyton floccosum* sind.

3. Fermentiertes Getreide zur Verwendung gemäß den Ansprüchen 1-2, worin die Pilzinfektion Onychomykose ist.

4. Fermentiertes Getreide zur Verwendung gemäß den Ansprüchen 1-3, worin das fermentierte Getreide in einer Zusammensetzung mit einem Penetrationsförderer vorhanden ist.

5. Fermentiertes Getreide zur Verwendung gemäß Anspruch 4, worin die Zusammensetzung weiter 1 bis 99 Gewichtsprozent Lösungsmittel umfasst.

6. Fermentiertes Getreide zur Verwendung gemäß den Ansprüchen 4-5, worin die Zusammensetzung weiter ein Verdickungsmittel umfasst.

7. Fermentiertes Getreide zur Verwendung gemäß den Ansprüchen 4-6, worin der Penetrationsförderer gewählt ist aus der Gruppe aus Ethanol, Glycerylmonoethylether, Monoglyceriden, Isopropylmyristat, Laurylalkohol und Derivaten, Isoprenoiden, beta-Cyclodextrinen, Dimethylsulfoxid, Phytantriol, Dimethylisosorbid, Polysorbaten, Fettsäuren mit C6-C14, N-Methylpyrrolidon, polyglycosylierten Glyceriden, 1-Dodecylazacycloheptan-2-on, Cyclopentadecalacton, 2-(n-Nonyl)-1,3-dioxolan, n-Octanol, Estern, Decanaldimethylacetal und ungesättigtem Phosphatidylcholin.

8. Fermentiertes Getreide zur Verwendung gemäß den Ansprüchen 4-7, worin die Zusammensetzung weiter Polyvinylalkoholpolymer umfasst.

9. Fermentiertes Getreide zur Verwendung gemäß den Ansprüchen 4-8, worin die Zusammensetzung weiter einen Osmoseprotektor in einem Anteil von 0,1 bis 5 Gewichtsprozent der Zusammensetzung umfasst.

10. Fermentiertes Getreide zur Verwendung gemäß den Ansprüchen 1-9, worin das fermentierte Getreide flüssig und in einem Applikator mit einem Pinsel, einem Behälter und einer Kappe vorhanden ist.

11. Fermentiertes Getreide zur Verwendung gemäß den Ansprüchen 1-10, worin das fermentierte Getreide auf einen Nagel und/oder eine Haut aufgetragen wird.

12. Nagelapplikator zur Verwendung in der Behandlung von Onychomykose, der einen Pinsel, einen Behälter, eine Kappe und eine flüssige antimykotische Zusammensetzung umfasst, die fermentiertes Getreide gemäß Anspruch 1 umfasst.

13. Applikator zur Verwendung gemäß Anspruch 12, wobei die antimykotische Zusammensetzung weiter einen Penetrationsförderer umfasst.

## Revendications

1. Graine de céréale fermentée destinée à être utilisée dans le traitement d'infections fongiques, dans laquelle la graine de céréale fermentée est du seigle fermenté par une espèce de Lactobacillus.

2. Graine de céréale fermentée destinée à être utilisée selon la revendication 1, dans laquelle la graine cible *Trichophyton rubrum, Trichophyton mentagrophytus* et *Epidermophyton floccosum.*

3. Graine de céréale fermentée destinée à être utilisée selon les revendications 1 à 2, dans laquelle l'infection fongique est l'onychomycose.

4. Graine de céréale fermentée destinée à être utilisée selon les revendications 1 à 3, dans laquelle la graine de céréale fermentée est présente dans une composition avec un améliorateur de pénétration.

5. Graine de céréale fermentée selon la revendication 4, dans laquelle la composition comprend en outre de 1 à 99 % en poids de solvant.

6. Graine de céréale fermentée destinée à être utilisée selon les revendications 4 à 5, dans laquelle la composition comprend en outre un agent épaississant.

7. Graine de céréale fermentée destinée à être utilisée selon les revendications 4 à 6, dans laquelle l'améliorateur de pénétration est choisi dans le groupe de l'éthanol, du glycéryl monoéthyl éther, de monoglycérides, de l'isopropylmyristate, d'alcool laurylique et de ses dérivés, d'isoprénoïdes, de béta-cyclodextrines, de sulfoxyde de diméthyle, de phytantriol, d'isosorbide de diméthyle, de polysorbates, d'acides gras en C6 à C14, de N-méthylpyrrolidone, de glycérides polyglycosylés, de 1-dodécylazacycloheptane-2-one, de cyclopentadécalactone, de 2-(n-nonyl)-1,3-dioxolanne, de n-octanol, d'esters, de décanal diméthyl acétal, de cyclopentadécalactone et de phosphatidylcholine insaturée.

8. Céréale de graine fermentée destinée à être utilisé selon les revendications 4 à 7, dans laquelle la composition comprend en outre un polymère de polyvinylalcool.

9. Graine de céréale fermentée destinée à être utilisée selon les revendications 4 à 8, dans laquelle la composition comprend en outre un protecteur d'osmose à un niveau de 0,1 à 5 % en poids de la composition.

10. Graine de céréale fermentée destinée à être utilisée selon les revendications 1 à 9, dans laquelle la graine de céréale fermentée est liquide et est présente dans un applicateur avec une brosse, un réservoir et une coiffe.

11. Graine de céréale fermentée destinée à être utilisé selon les revendications 1 à 10, dans laquelle la graine de céréale fermentée est appliquée à un ongle et/ou une peau.

12. Applicateur pour ongle destiné à être utilisé dans le traitement de l'onychomycose comprenant une brosse, un réservoir, une coiffe et une composition antifongique liquide comprenant la graine de céréale fermentée selon la revendication 1.

13. Applicateur destiné à être utilisé selon la revendication 12, dans lequel la composition antifongique comprend en outre un améliorateur de pénétration.
